# EUROPEAN PATENT SPECIFICATION

(11) **EP 1 799 308 B1**
(45) Date of publication and mention of the grant of the patent: **04.06.2014**
(21) Application number: 05807605.0
(22) Date of filing: 11.10.2005
(51) Int. Cl.: A61Q 5/02, A61Q 5/12, A61K 8/24, A61K 8/73, A61K 8/894, A61K 8/81, A61K 8/891

(54) **CONDITIONING SHAMPOOS WITH DETERGENT SOLUBLE SILICONES**
KONDITIONIERSHAMPOO MIT IN REINIGUNGSMITTELN LÖSLICHEN SILIKONEN
SHAMPOOINGS TRAITANTS A SILICONES SOLUBLES DETERGENTES

(30) Priority: 13.10.2004 US 964255
(43) Date of publication of application: 27.06.2007
(73) Proprietor: Colgate-Palmolive Company, New York NY 10022-7499 (US)
(72) Inventor: KOZUBAL, Cheryl, Westampton, New Jersey 08060 (US); HARTNETT, Donna, Belle Mead, New Jersey 08502 (US); FEI, Lin, Kendall Park, New Jersey 08824 (US); DUGDALE, Julia, Cranbury, New Jersey 08512 (US); REICH, Charles, Highland Park, New Jersey 08904 (US)
(74) Representative: Jenkins, Peter David
(86) International application number: PCT/US2005/036313
(87) International publication number: WO 2006/044296

(56) References cited:
- EP-A- 1 136 066
- WO-A-97/04743
- US-A- 5 684 112
- US-A- 6 022 547

## Description

### Field of the Invention

This invention relates to improved conditioning shampoos that may be made without stabilizing agents for silicones.

### Background of the Invention

A variety of conditioning shampoos (also called "2-in-1 shampoos") have been sold and/or described in the patent literature. These products characteristically include an insoluble silicone such as dimethicone in combination with some type of stabilizing agent. The stabilizing agents that have been used have included, for example, ethylene glycol distearate (which is also a pearlizing agent), long chain alcohols such as those having C20-40 carbons (commonly referred to as "Unilin" technology), behenyl alcohol, selected acrylates such as ACULYN-33, and other systems such as cetearyl alcohol mixtures. Some of the stabilizers require heat in the manufacturing processes and others made be made at room temperature.

U.S. Patent Number 5,945,093 describes an improved stable conditioning shampoo imparts improved physical and cosmetic properties to the hair such as wet and dry comb, wet and dry feel, gloss, static control and manageability, yet does not cause build-up and does not contain any polyhydric compounds. The shampoo includes an anionic surfactant, a water soluble cationic surfactant, a water-insoluble conditioning agent, and a suspending agent where the suspending agent is a mixture of a cellulose derivative and a PVM/MA decadiene cross polymer. The conditioning shampoo is stable for extended periods.

U.S. Patent Number 5,932,203 describes conditioning shampoo compositions which comprise (A) an anionic surfactant component selected from the group consisting of anionic surfactants, zwitterionic or amphoteric surfactants having an attached group that is anionic at the pH of the composition (B) an organic, cationic, hair conditioning polymer; (C) a selected water insoluble, synthetic ester; and (D) water.

U.S. Patent Number 6,287,546 describes improved stabilized shampoo compositions containing siloxysilicate materials commonly referred to as MQ resins, wherein the stabilizers are selected from (i) long chain fatty alcohols with greater than 14 carbons; (ii) acrylates/steareth-20 methacrylate copolymer; acrylates copolymer; and acrylates/C10-30 alkyl acrylate cross polymer; and (iii) selected N,N-disubstituted phthalamic acids and their ammonium salts.

Of course the use of soluble silicones can be complex if a product is to be obtained that does not rinse the soluble silicone down the drain during shampooing. Thus, there remains a need for conditioning shampoos that can deliver a higher degree of conditioning. There is also a need for conditioning shampoos that can be made in part or in whole without the need for stabilizing agents required for insoluble silicone materials. Yet another object is to create conditioning shampoos that can use soluble silicones and still achieve good conditioning.

### Brief Summary of the Invention

The present invention provides a conditioning shampoo according to claim 1. Preferred features are defined in the dependent claims.

The conditioning shampoos of this invention comprise:
(a) 6-19 weight % of an anionic surfactant or mixtures of two or more anionic surfactants;
(b) 0.025-3 weight % of a cationic deposition polymer, wherein the cationic deposition polymer is at least one polymer chosen from polyquaternium-7 and polyquaternium-10;
(c) 0.4-5 weight % of a detergent soluble, water dispersible silicone copolyol comprising PEG-12 dimethicone having a molecular weight of about 2300;
(d) a non-zero amount up to 5 weight % of a cocamidopropyl betaine surfactant; and
(e) water;
wherein all amounts are based on the weight of the entire composition.

In addition to these ingredients, one or more optional ingredients selected from the group consisting of the following list may be included:
(f) 0-10 weight % (particularly 0.5-10 weight %) of a co-surfactant or mixtures of more than one co-surfactant selected from the group consisting of amphoteric, nonionic and zwitterionic surfactants and mixtures thereof;
(g) 0-5 weight % of a pearlizer (including ethylene glycol distearate);
(h) 0-5 weight % of a salt such as NaCl as a viscosity modifier;
(i) 0-0.5 weight % of a chelating agent such as EDTA;
(j) 0-1 weight % of a buffering agent (which may also have some chelating properties) such as sodium dihydrogen phosphate monohydrate;
(k) 0-5.0 weight % fragrance (particularly 0.2-2.0%);
(1) 0-1 weight % of a pH adjuster (such as citric acid);
(m) 0-5 weight % of a coloring agent;
(n) 0-8 weight % of an insoluble silicone;
(o) 0-5 weight % of a silicone resin;
(p) 0-10 weight % of a stabilizing agent for the insoluble silicone and/or the silicone resin (particularly 0-5.0%); and
(q) 0-2 weight % of a preservative.
Note that for ingredients (a) and (b), these ingredients may be added as aqueous solutions (for example, having 8-90% of the active ingredient). For optional ingredients (f) and (g), they may be added as a pre-formed aqueous solution or suspension. For optional ingredients (h), (i), (j), (l), (m), (p) and (q) these ingredients may be added as aqueous solutions. For optional ingredients (n) and (o), these ingredients may be added as pre-formed stabilized emulsions.

### Detailed Description of the Invention

The shampoos of this invention may be made as clear, translucent or opaque products which are colored or colorless. In addition, the compositions of this invention may be made with soluble silicones only and without the need for any stabilizers for the silicones, or they may be made by supplementing the system with a portion of insoluble silicone with a conventional stabilizing system for those silicones.

Additionally, in a special embodiment, an MQ resin is used in the formulation to form a 3-in-1 product with special styling capabilities.

The anionic surfactants useful in the compositions of the invention include those familiar to skilled practioners. The anionic surfactant of component (a) will generally be present at a level from 6 weight % to 19 weight %, with an amount of about 7-17 weight % being of particular interest.

Anionic detersive surfactants useful herein include those that are disclosed in U.S. Patent Number 5,573,709. Examples of the anionic surfactants useful in this invention include alkyl and alkyl ether sulfates, particularly those having from 7-25 carbons. Specific examples of alkyl ether sulfates which may be used in the present invention are sodium and ammonium salts of lauryl sulfate, lauryl ether sulfate, coconut alkyl triethylene glycol ether sulfate; tallow alkyl triethylene glycol ether sulfate, and tallow alkyl hexaoxyethylene sulfate. A particular group of anionic surfactants includes alkyl ether sulfates comprising a mixture of individual compounds, said mixture having an average alkyl chain length of from about 12 to about 16 carbon atoms and an average degree of ethoxylation of from about 1 to about 6 moles of ethylene oxide.

Another suitable class of anionic detersive surfactants are the alkyl sulfuric acid salts. Important examples are the salts of an organic sulfuric acid reaction product of a hydrocarbon of the methane series, including iso-, neo-, ineso-, and n-paraffins, having about 8 to about 24 carbon atoms, preferably about 12 to about 18 carbon atoms and a sulfonating agent, for example, SO₃,H₂SO₄, oleum (fuming sulfuric acid) obtained according to known sulfonation methods, including bleaching and hydrolysis. Preferred are alkali metal and ammonium sulfated C₁₂₋₁₈ n-paraffins.

Additional examples of synthetic anionic detersive surfactants which come within the terms of the present invention are the olefin sulfonates, the beta-alkyloxy alkane sulfonates, and the reaction products of fatty acids esterified with isethionic acid and neutralized with sodium hydroxide, as well as succinamates. Specific examples of succinamates include disodium N-octadecyl sulfofosuccinamate; tetrasodium N-(1,2-dicarboxyethyl)-N-octadecylsulfosuccinainate; diamyl eater of sodium sulfosuccinic acid; dihexyl ester of sodium sulfosuccinic acid; and dioctyl esters of sodium sulfosuccinic acid.

Many additional synthetic anionic surfactants are described in McCutcheon's Emulsifiers and Detergents, 1989 Annual, published by M. C. Publishing Co. Finally, U.S. Patent Number 3,929,678, Laughlin et al., discloses many other anionic as well as other surfactant types.

A particular group of anionic detersive surfactants for use in the present shampoo compositions include one or more members selected from the group consisting of ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, and mixtures of two or more of the foregoing.

A particular anionic component is a combination of 5-20 weight % of ammonium lauryl sulfate and 5-20 weight % of ammonium lauryl ether sulfate.

The shampoo compositions of the present invention comprise a cationic polymer particularly an organic polymer, as a deposition aid polymer. Such cationic polymers should also be physically and chemically compatible with the essential components described herein, or should not otherwise unduly impair product stability, aesthetics or performance. Cationic deposition polymers polyquaternium-7 and/or polyquaternium-10 are included in the compositions of the present invention.

The concentration of the organic, cationic, conditioning polymer of the shampoo composition should be generally in the range from 0.025% to 3%, preferably from about 0.05% to about 2%, more preferably from about 0.1% to about 1%, by weight of the shampoo composition.

The cationic deposition polymer contains cationic nitrogen-containing moieties such as quaternary ammonium or cationic protonated amino moieties. The cationic protonated amines can be primary, secondary, or tertiary amines (preferably secondary or tertiary), depending upon the particular species and the selected pH of the shampoo composition. The average molecular weight of the cationic conditioning polymers is between about 10 million and about 5,000, preferably at least about 100,000, more preferably at least about 200,000, but preferably not more than about 2 million, preferably not more than about 1.5 million. The polymers also have a cationic charge density ranging from about 0.2 meq/gm to about 7 meq/gm, preferably at least about 0.4meq/gm, more preferably at least about 0.6meq/gm, but also preferably less than about 5meq/gm, more preferably less than about 2 meq/gm, at the pH of intended use of the shampoo composition, which pH will generally range from about pH 3 to about pH 9, preferably between about pH 4 and about pH7.

Any anionic counterions can be used in association with the cationic deposition polymers so long as the polymers remain soluble in water, in the shampoo composition, or in a coacervate phase of the shampoo composition, and so long as the counterions are physically and chemically compatible with the essential components of the shampoo composition or do not otherwise unduly impair product performance, stability or aesthetics. Non-limiting examples of such counterions include halides (for example, chlorine, fluorine, bromine, iodine), sulfate and methylsulfate).

The cationic nitrogen-containing moiety of the cationic polymer is generally present as a substituent on all, or more typically on some, of the monomer units thereof. Thus, the cationic polymer for use in the shampoo composition includes homopolymers, copolymers, terpolymers, and so forth, of quaternary ammonium or cationic amine-substituted monomer units, optionally in combination with non-cationic monomers referred to herein as spacer monomers. Non-limiting examples of such polymers are described in the CTFA Cosmetic Ingredient Dictionary, 3rd edition, edited by Estrin, Crosley, and Haynes (The Cosmetic, Toiletry, and Fragrance Association, Inc., Washington, D.C. (1982)).

Non-limiting examples of additional suitable cationic polymers include copolymers of vinyl monomers having cationic protonated amine or quaternary ammonium functionalities with water soluble spacer monomers such as acrylamide, methacrylamide, alkyl and dialkyl acrylamides, alkyl and dialkyl methacrylamides, alkyl acrylate, allyl methacrylate, vinyl caprolactone or vinyl pyrrolidone. The alkyl and dialkyl substituted monomers preferably have from C₁ to C₇ alkyl groups, more preferably from C₁ to C₃ alkyl groups. Other suitable spacer monomers include vinyl esters, vinyl alcohol (made by hydrolysis of polyvinyl acetate), maleic anhydride, propylene glycol, and ethylene glycol.

Suitable additional cationic protonated amino and quaternary ammonium monomers, for inclusion in the cationic polymers of the shampoo composition herein, include vinyl compounds substituted with dialkylaminoalkyl acrylate, dialkylaminoalkyl methacrylate, monoalaylaminoalkyl acrylate, monoalkylaminoalkyl methacrylate, trialkyl methacryloxyalkyl ammonium salt, trialkyl acryloyalyl ammonium salt, diallyl quaternary ammonium salts, and vinyl quaternary ammonium monomers having cyclic cationic nitrogen-containing rings such as pyridinium, imidazolium, and quaternized pyrrolidone, e.g., alkyl vinyl imidazolium, alkyl vinyl pyridinium, alkyl vinyl pyrrolidone salts. The alkyl portions of these monomers are preferably lower alkyls such as C₁, C₂ or C₃ alkyls.

Suitable amine-substituted vinyl monomers for use herein include dialkylaminoalkyl acrylate, dialkylaminoalkyl methacrylate, dialkylaminoalkyl acrylamide, and dialkylaminoalkyl methacrylamide, wherein the alkyl groups are preferably C₁ -C₇ hydrocarbyls, more preferably C₁ -C₃, alkyls.

Other suitable additional cationic polymers for use in the shampoo composition include copolymers of 1-vinyl-2-pyrrolidone and 1-vinyl-3-methylimidazolium salt (for example, chloride salt) (referred to in the industry by the Cosmetic, Toiletry, and Fragrance Association, "CTFA", as Polyquaternium-16), such as those commercially available from BASF Wyandotte Corp. (Parsippany, N.J.) under the LUVIQUAT trade name (for example, LUVIQUAT FC 370); copolymers of 1-vinyl-2-pyrrolidone and dimethylaminoethyl methacrylate (referred to in the industry by CTFA as Polyquaternium-11) such as those commercially available from ISP Corporation (Wayne, N.J.) under the GAFQUAT trade name (for example, GAFQUAT 755N); cationic diallyl quaternary ammonium-containing polymers, including, for example, dimethyldiallylammonium chloride homopolymer and copolymers of acrylamide and dimethyldiallylammonium chloride, referred to in the industry (CTFA) as Polyquaternium-6 and Polyquaternium-7, respectively; and mineral acid salts of aminoalkyl esters of homopolymers and copolymers of unsaturated carboxylic acids having from 3 to 5 carbon atoms, as described in U.S. Patent Number 4,009,256.

Other suitable additional cationic polymers for use in the shampoo composition include polysaccharide polymers, such as cationic cellulose derivatives and cationic starch derivatives. Suitable cationic polysaccharide polymers include those which conform to the formula: 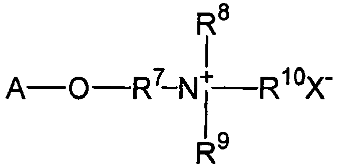 wherein A is an anhydroglucose residual group, such as a starch or cellulose anhydroglucose residual; R⁷ is an alkylene oxyalkylene, polyoxyalkylene, or hydroxyalkylene group, or combination thereof, R⁸, R⁹, and R¹⁰ independently are alkyl, aryl, alkylaryl, arylalkyl, alkoxyalkyl, or alkoxyaryl groups, each group containing up to about 18 carbon atoms, and the total number of carbon atoms for each cationic moiety (i.e., the sum of carbon atoms in R⁸, R⁹ and R¹⁰) preferably being about 20 or less; and X is an anionic counterion as described in hereinbefore.

Cationic cellulose polymers included in the compositions of the present invention are those polymers available from Amerchol Corp. (Edison, N.J.,) in their Polymer JR and LR series of polymers, as salts of hydroxyethyl cellulose reacted with trimethyl ammonium substituted epoxide, referred to in the industry (CTFA) as Polyquaternium-10; and/or the polymeric quaternary ammonium salts of acrylamide and dimethyl diallyl ammonium chloride monomers, referred to in the industry (CTFA) as Polyquaternium-7, available from Nalco Company (Naperville, IL) under the trade name Merquat 550.

Other suitable additional cationic polymers include cationic guar gum derivatives, such as guar hydroxypropyltrimonium chloride, specific examples of which include the Jaguar series commercially available from Celanese Corporation. Other suitable cationic polymers include quaternary nitrogen-containing cellulose ethers, some examples of which are described in U.S. Patent Number 3,962,418. Other suitable cationic polymers include copolymers of etherified cellulose, guar and starch, some examples of which are described in U.S. Patent Number 3,958,581.

The water dispersible silicone copolyol comprises PEG-dimethicone having a molecular weight of about 2300. With regard to the soluble silicones used in the compositions of this invention, the silicones are soluble in the aqueous surfactant-containing systems described above and are generally classified as dimethicone copolyols having a molecular weight less than 100,000 daltons, particularly in the range of 1,000-10,000 daltons and, more particularly, in the range of 2,000-4,000 daltons. PEG-12 dimethicone (a detergent soluble, water dispersible silicone copolyol with a molecular weight of about 2300 and sold as DC-5324 by Dow Corning) is included in the compositions of the present invention.

The compositions of this invention may be made using the detergent soluble silicone as the only silicone conditioning component.

A particular embodiment of interest is one that further comprises a co-surfactant, for example, 0-10 weight % (particularly 0.5-10 weight %) of a co-surfactant or mixtures of more than one co-surfactant selected from the group consisting of amphoteric, nonionic and zwitterionic surfactants and mixtures thereof. Nonionic detersive surfactants which can be used in the invention include those selected from the group described in U.S. Patent No. 4,741,855 to Grote et al including:
(i) polyethylene oxide condensates of alkyl phenols wherein the alkyl portion of the alkyl phenol has 6-12 carbons and may be straight chain or branched and the ethylene oxide portion is present in an amount of 10-60 moles of ethylene oxide per mole of alkyl phenol;
(ii) condensation products of ethylene oxide with a product resulting from the reaction of propylene oxide and ethylene diamine varied according to the hydrophobic/hydrophilic balance desired (for example, compounds containing from 40-80% polyoxyethylene by weight and having a molecular weight of from 5,000-11,000 resulting from the reaction of ethylene oxide groups with a hydrophobic base constituted of the reaction product of ethylene diamine and excess propylene oxide, wherein the base has a molecular weight of 2500-3,000);
(iii) condensation products of C8-18 straight or branched chain aliphatic alcohols with ethylene oxide (for example, coconut alcohol ethylene oxide condensate with 10-30 moles of ethylene oxide per mole of coconut alcohol wherein the coconut fraction has 10-14 carbon atoms);
(iv) long chain tertiary amine oxides of formula (R¹)(R²)(R³)-N →O, wherein R¹ is an C8-18 alkyl, alkenyl or monohydroxy alkyls; which has from 0-10 ethylene moieties and from 0-1 glyceryl moiety; and R² and R³ may be the same or different and are each independently selected from the group consisting of C1-3 alkyls with 0-1 hydroxy group; the arrow in the structure is a conventional representation of a semi-polar bond (examples of suitable long chain tertiary amine oxides including cocamidopropylamine oxide and lauramine oxide);
(v) long chain tertiary phosphine oxides of Formula: R⁴ R⁵ R⁶ P→O where R⁴ contains a C8-18 alkyl, alkenyl or monohydroxyalkyl radical; 0-10 ethylene oxide moieties and 0-1 glyceryl moiety; and R⁵ and R⁶ are each independently C1-3 alkyl or monohydroxyalkyl with the arrow in the formula being a conventional representation of a semi-polar bond; and
(vi) long chain dialkyl sulfoxides containing one short chain alkyl or hydroxy alkyl radical of 1-3 carbons (particularly methyl) and one long hydrophobic chain having a C8-20 alkyl, alkenyl, hydroxy alkyl or keto alkyl group, with 0-10 ethylene oxide moieties and 0-1 glyceryl moiety.

Other representative nonionic surfactants include esters of polyols or sugars; fatty acid alkanolamides having 7-25 carbons; pyrrolidones; and the condensation products of ethylene oxide and C7-C25 long chain amides. These nonionic surfactants, as well as numerous others not cited herein, are well known in the art and are fully described in the literature, such as McCutcheon's Detergents and Emulsifiers.

In particular, a nonionic alkanolamide can be included in the composition to provide foam stability. The alkanolamide can be included in an amount of 0% to about 5% by weight of the composition. Accordingly, suitable alkanolamides include, but are not limited to, those known in the art of hair care formulations, such as cocamide MEA, cocamide DEA, soyamide DEA, lauramide DEA, oleamide MIPA, stearamide MEA, myristamide MEA, lauramide MEA, capramide DEA, ricinoleamide DEA, myristamide DEA, stearamide DEA, oleylamide DEA, tallowamide DEA, lauramide MEA, tallowamide MEA, isostearamide DEA, isostearamide MEA, and combinations thereof where DEA means diethanolamide; MEA means monoethanolamide; and MIPA means monoisopropyl amide.

In the compositions of the present invention, amphoteric surfactants may also be included. Examples of amphoteric detersive surfactants which can be used in the compositions of the present invention are those which are broadly described as derivatives of aliphatic secondary and tertiary amines in which the aliphatic substituent contains from about 8 to 18 carbon atoms and contains an anionic water solubilizing group, for example, carboxy, sulfonate, sulfate, phosphate, or phosphonate. Examples of compounds falling within this definition are sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. Patent Number. 2,658,072, N-higher alkyl aspartic acids such as those produced according to the teaching of U.S. Patent Number 2,438,091, and the products sold under the trade name "MIRANOL" as described in U.S. Patent Number 2,528,378.

In the present invention, a cocamidopropyl betaine surfactant is included in the composition. Additional zwitterionics such as betaines can also useful. Examples of suitable betaines include the high alkyl betaines, such as cocodimethyl carboxymethyl betaine, cocobetaine, lauryl amidopropyl betaine, oleyl betaine, lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, and lauryl bis-(2-hydroxypropyl)alpha-carboxyethyl betaine. The sulfobetaines may be represented by cocodimethyl sulfopropyl betaine, stearyl dimethyl sulfopropyl betaine, lauryl dimethyl sulfoethyl betaine, lauryl bis-(2-hydroxyethyl) sulfopropyl betaine and the like; amidobetaines and amidosulfobetaines, wherein the RCONH(CH₂)₃ radical is attached to the nitrogen atom of the betaine are also useful in this invention.

A specific betaine of interest is a non-zero amount up to 5 weight % of cocoamidopropyl betaine.

With regard to insoluble silicones, such insoluble silicones are characteristically a nonvolatile, nonionic silicone hair conditioning agent which is insoluble in the shampoo compositions hereof. The silicone hair conditioning agent is intermixed in the shampoo composition so as to be in the form of dispersed, insoluble particles, or droplets. The silicone hair conditioning agent comprises a nonvolatile, insoluble, silicone fluid and optionally comprises a silicone gum which is insoluble in the shampoo composition as a whole but is soluble in the, silicone fluid. The silicone hair conditioning agent can also comprise other ingredients, such as a silicone resin to enhance deposition efficiency. These insoluble silicones have particular viscosities of from about 200 to about 2,000,000 centistokes at 25 degrees C., more particularly from about 1,000 to about 1,800,000, even more particularly from about 10,000 to about 1,500,000 cst. The viscosity can be measured by means of a glass capillary viscometer as set forth in Dow Coming Corporate test method CTM0004, July 20, 1970.

The insoluble silicone hair conditioning agent may be used in the shampoo compositions hereof at levels of from about 0 weight % to about 8 weight % based on the weight of the composition, particularly from about 0.5 weight % to about 4 weight%.

Suitable insoluble, nonvolatile silicone fluids include polyalkyl siloxanes, polyaryl siloxanes, polyalkylaryl siloxanes, polyether siloxane copolymers, and mixtures thereof. Other insoluble, nonvolatile silicones fluids having hair conditioning properties can also be used. The term "nonvolatile" as used herein shall mean that the silicone material exhibits very low or no significant vapor pressure at ambient conditions, as is understood by those in the art. The term "silicone fluid" shall mean flowable silicone materials having a viscosity of less than 1,000,000 centistokes at 25 degrees C. Generally, the viscosity of the fluid will be between about 5 and 1,000,000 centistokes at 25 degrees C., preferably between about 10 and about 100,000 centistokes.

The nonvolatile polyalkylsiloxane fluids that may be used include, for example, polydimethyl siloxanes. These siloxanes are available, for example, from the General Electric Company in their Viscasil® and SF 96 series, and from Dow Coming in their Dow Coming 200 series.

The polyalkylaryl siloxane fluids that may be used, also include, for example, polymethylphenylsiloxanes. These siloxanes are available, for example, from the General Electric Company as SF 1075 methyl phenyl fluid or from Dow Coming as 556 Cosmetic Grade Fluid.

The polyether siloxane copolymers that may be used include, for example, a polypropylene oxide modified polydimethylsiloxane (for example, Dow Coming DC-1248) although ethylene oxide or mixtures of ethylene oxide and propylene oxide may also be used. The ethylene oxide and polypropylene oxide level must be sufficiently low to prevent solubility in water and the composition hereof.

References disclosing suitable silicone fluids include U.S. Patent Number 2,826,551, Geen; U.S. Patent Number 3,964,500, Drakoff; U.S. Patent Number 4,364,837, Pader; U.S. Patent Number 5,573,709, Wells; British Patent 849,433, Woolston; and PCT Patent Application WO 93/08787.

Another silicone material that can be especially useful in the silicone conditioning agents is insoluble silicone gum. The term "silicone gum", as used herein, means polyorganosiloxane materials having a viscosity at 25 degrees C of greater than or equal to 1,000,000 centistokes. Silicone gums are described in U.S. Patent Number 4,152,416, Spitzer et al; and in Noll, Walter, Chemistry and Technology of Silicones, New York: Academic Press 1968. Also describing silicone gums are General Electric Silicone Rubber Product Data Sheets SE 30, SE 33, SE 54 and SE 76. The "silicone gums" will typically have a mass molecular weight in excess of about 200,000, generally between about 200,000 and about 1,000,000. Specific examples include polydimethylsiloxane, (polydimethyl siloxane) (methylvinylsiloxane) copolymer, poly(dimethyl siloxane) (dipheayl siloxane)(methylvinylsiloxane) copolymer and mixtures thereof.

Another optional ingredient that can be included in the shampoo is silicone resin. Silicone resins are highly crosslinked polymeric siloxane systems. The crosslinking is introduced through the incorporation of trifunctional and tetrafunctional silanes with monofunctional or difunctional, or both, silanes during manufacture of the silicone resin. As is well understood in the art, the degree of crosslinking that is required in order to result in a silicone resin will vary according to the specific silane units incorporated into the silicone resin. The ratio of oxygen atoms to silicon atoms is indicative of the level of crosslinking in a particular silicone material. Silanes used in the manufacture of silicone resins include monomethyl-, dimethyl-, trimethyl-, monophenyl-, diphenyl-, methylphenyl-, monovinyl-, and methylvinyl-chlorosilanes, and tetrachlorosilane, with the methyl-substituted silanes being most commonly utilized. Preferred resins are offered by General Electric as GE SR399, SS4230 and SS4267. Commercially available silicone resins may be supplied in a dissolved form in a low viscosity volatile or nonvolatile silicone fluid.

Background material on silicones including sections discussing silicone fluids , gums, and resins, as well as manufacture of silicones, can be found in Encyclopedia of Polymer Science and Engineering, Volume 15, Second Edition, pages 294-308, John Wiley & Sons, Inc., 1989.

Silicone materials and silicone resins in particular, can conveniently be identified according to a shorthand nomenclature system well known to those skilled in the art as "MDTQ" nomenclature. Under this system, the silicone is described according to presence of various siloxane monomer units which make up the silicone. Briefly, the symbol M denotes the monofunctional unit (CH₃)₃ SiO_{0.5} ; D denotes the difunctional unit (CH₃)₂ SiO; T denotes the trifunctional unit (CH₃)SiO_{1.5} ; and Q denotes the quadri- or tetra-functional unit SiO₂. Primes of the unit symbols, D', T', and Q' denote substituents other than methyl, and must be specifically defined for each occurrence. Typical alternate substituents include groups such as vinyl, phenyls, amines, hydroxyls, etc. The molar ratios of the various units, either in terms of subscripts to the symbols indicating the total number of each type of unit in the silicone (or an average thereof) or as specifically indicated ratios in combination with molecular weight complete the description of the silicone material under the MDTQ system. Higher relative molar amounts of T, Q, T' and/or Q' to D, D', M and/or or M' in a silicone resin is indicative of higher levels of crosslinking. As discussed before, however, the overall level of crosslinking can also be indicated by the oxygen to silicon ratio.

The silicone resins for use herein which are preferred are MQ, MT, MTQ, MQ and MDTP resins. Thus, the preferred silicone substituent is methyl. Especially preferred are MQ resins wherein the M:Q ratio is from about 0.5:1.0 to about 1.5:1.0 and the average molecular weight of the resin is from about 1000 to about 100,000.

More particular MQ resins suitable for use with this invention may be represented by Formula IA: 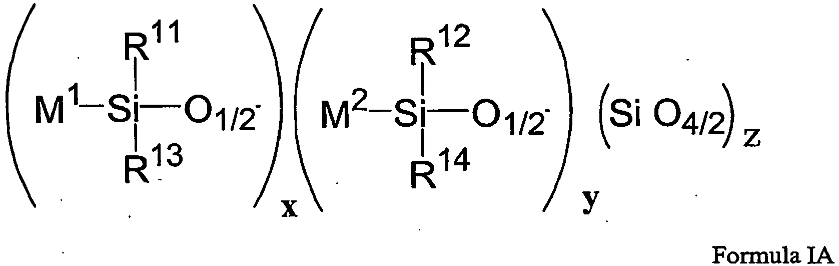
wherein R¹¹, R¹², R¹³, and R¹⁴ are each independently selected from the group consisting of phenyl and C1-C12 branched and unbranched hydrocarbons, particularly C1-C12 branched and unbranched alkyl, more particularly branched and unbranched C1-C5 alkyl and especially methyl;
M¹ and M² are each independently from the group consisting of:
   (a) hydrogen,
   (b) phenyl,
   (c) phenethyl,
   (d) a polyether of Formula II:

      -H₂C - (CH₂)ₙ - (O-CH(R¹⁵)-CH₂)ᵤ - (OCH₂-CH₂)ᵥ-OR¹⁶ Formula II

      where n is a number from 1-20 and the -(CH₂)- chain may optionally contain 1 or 2 unsaturations; u and v are integers each independently selected from 0-20, provided that u +v ≥ 1; R¹⁵ is selected from C1-C20 alkyl; and R¹⁶ is selected from the group consisting ofH, -CH₃ and -C(O)CH₃); and
   (e) C1-C24 branched and unbranched hydrocarbons optionally substituted by a halogen substituted C1-C3 hydrocarbon radical, with a particular value being C1-C24 alkyl, especially methyl; and wherein (x + y)/z is a number in the range of 0.5 and 1.5, and is preferably equal to 1; and the values for R¹¹, R¹², R¹³, R¹⁴, x, y, z, M¹ and M² are selected to so that the MQ resin is a liquid having a viscosity of 1.0 x 10³ - 1 x 10⁶ centipoise, such as 1.5 x 10³ - 1 x 10⁶ centipoise.

A very particular type of MQ resin is described in U.S. Patent Number 6,294,159 assigned to the same owner as this invention. In this very particular type of MQ resin, the MQ Resin structure is of Formula IA when x and y are the same may be represented by Formula IB: 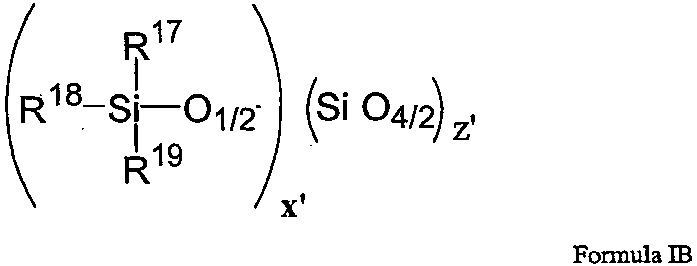 wherein R¹⁷ and R¹⁹ are each independently selected from the same group as defined for R¹¹, R¹², R¹³ and R¹⁴ of Formula IA; R¹⁸ is selected from the same group as described for M¹ and M²; and x'/z' is a value between 0.5 and 1.5.

An even more particular MQ useful in the invention is a liquid trimethylsiloxysilicate polymer, especially with an M:Q ratio of 1 (for example, a resin obtained from General Electric Company, Waterford, New York as SR 399).

Examples of particular insoluble silicones include, dimethicone, cyclomethicone, trimethyl silyl amodimethicone, phenyl trimethicone, trimethyl siloxy silicate, polyorganosiloxane, polyalkylsiloxane, polyarylsiloxane, polyalkylarylsiloxane, and polyestersiloxane copolymers.

If insoluble silicones are used a suitable amount of a stabilizing agent must also be included so as to stabilize the insoluble silicone. Examples of such stabilizers include one or more members selected from the group consisting of the following members which are selected so that the final amount of stabilizer added is in the range of 0.1-10.0 weight%:
(a) long chain fatty alcohols with greater than 14 carbons, for example C20-40, and mixtures of such long chain fatty alcohols (for example, a C>14 alcohol and ethene homopolymer PETROLITE C-7138 from Petrolite Corporation, St. Louis, Mo.).
(b) acrylates/steareth-20 methacrylate copolymer (for example, ACULYN® 22, from Rohm & Haas, Philadelphia, Pa.); and acrylates copolymer (for example, acrylates copolymer (ACULYN® 33); ACUSOL®-445, -810, and -830; ACRYSOL® ASE 75 from Rohm & Haas; AQUA SF-1 Carbopol® from Noveon); and acrylates/C10-30 alkyl acrylate crosspolymer (PEMULEN™ polymeric emulsifiers from BF Goodrich Company, Brecksville, Ohio, particularly products designated as TR-1 and TR-2). (For the acrylates copolymer (ACULYN® 33) product (having a pH in the range of 2.1-3.5), a neutralization step is performed with sodium phosphate (such as disodium phosphate), sodium hydroxide or a cosmetically acceptable organic amine to increase the pH to approximately 6.5.);
(c) agents described in U.S. Patent Number 5,015,415 especially N,N-disubstituted phthalamic acids and their ammonium salts selected from the group consisting of Formula III: 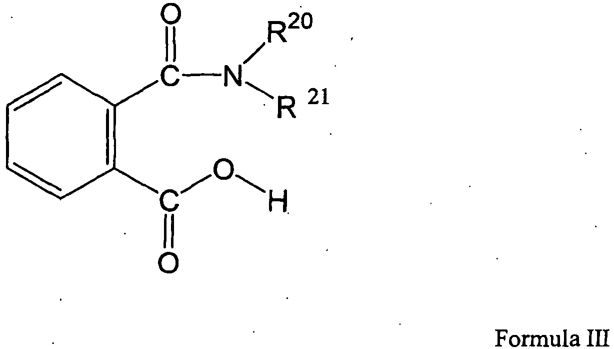
where R²⁰ and R²¹ may be the same or different and are each selected from the group consisting of C10-C40 straight and branched chain alkyl groups, and C10-C40 straight and branched arylalkyl groups (for example, where R²⁰ and R²¹ are the same and are each selected from the group consisting of stearyl and hydrogenated tallow such as STEPAN SAB-2 and STEPAN TAB®-2 from Stepan Company, Northfield, Ill.).
The stabilizing agents should be of a grade and purity acceptable for cosmetic use or purified as needed to be cosmetically acceptable. A further discussion of some of these agents may be found in U.S. Patent Number 5,015,415 to Goze et al and U.S. Patent Number 6,287,546 to Reich, et al.

In general, the compositions of this invention which contain only the soluble silicones as the only conditioning silicone component will be clear or translucent unless an opacifying agent is added. Other optional ingredients may be included, such as insoluble silicones, stabilizers and/or pearlizers, which may opacify the shampoo formulation.

Selected general formulations of the invention included can be made following the methods that are described in the Example section of this document.

Examples A to D and F, H-I are included below for information, and are not in accordance with the present invention

Examples A-D are of shampoo compositions having only the soluble silicone material and include:

**Formulation A**

| Water | Q.S. |
|---|---|
| Guar Hydroxypropyl Trimonium Chloride | 0.1-0.4% |
| Polyquaternium-7 | 0.2-0.5% |
| Tetrasodium EDTA | 0.05-0.1% |
| Sodium Phosphate Monobasic | 0.1-0.5% |
| Sodium Lauryl Ether Sulfate | 8-15% |
| PEG-12 Dimethicone (DC 5324) | 0.2-1.5% |
| Cocomonoethanolamide | 1-4% |
| Ethylene Glycol Distearate | 0.5-5% |
| Preservative | 0.1-0.25% |
| Fragrance | 0.4-1.0% |

**Formulation B**

| Water | Q.S. |
|---|---|
| Polyquaternium-10 | 0.1-0.5% |
| Tetrasodium EDTA | 0.1-0.2% |
| Sodium Phosphate Monobasic | 0.05-0.5% |
| Sodium Lauryl Sulfate | 9-12% |
| PEG-12 Dimethicone (DC 5324) | 0.2-1% |
| Cocodiethanolamide | 1-5% |
| Preservative | 0.1-0.25% |
| Fragrance | 0.4-2.0% |

**Formulation C**

| Water | Q.S. |
|---|---|
| Polyquaternium-7 | 0.05-1% |
| Tetrasodium EDTA | 0.1-0.2% |
| Sodium Phosphate Monobasic | 0.05-0.5% |
| Ammonium Lauryl Sulfate | 5-8% |
| Ammonium Lauryl Ether Sulfate | 3-7% |
| Lauryl/myristylamidopropyl Dimethylamine Oxide | 1-3% |
| PEG-12 Dimethicone (DC 5324) | 0.1-5% |
| Cocomonoethanolamide | 0.5-2.5% |
| Behenyl Alcohol | 0.5-2% |
| Preservative | 0.1-0.25% |
| Fragrance | 0.4-2.0% |

**Formulation D**

| Water | Q.S. |
|---|---|
| Polyquaternium-7 | 0.05-1% |
| Tetrasodium EDTA | 0.1-0.2% |
| Sodium Phosphate Monobasic | 0.05-0.5% |
| Ammonium Lauryl Sulfate | 5-8% |
| Ammonium Lauryl Ether Sulfate | 3-7% |
| Lauryl/myristylamidopropyl Dimethylamine Oxides | 1-3% |
| PEG-12 Dimethicone (DC 5324) | 0.1-5% |
| Cocomonoethanolamide | 0.5-2.5% |
| Cetyl/Stearyl Alcohol | 0.5-2% |
| Preservative | 0.1-0.25% |
| Fragrance | 0.4-2.0% |

Examples E-F are of shampoo compositions having a combination of soluble and insoluble silicones and include:

**Formulation E**

| Water | Q.S. |
|---|---|
| Guar Hydroxypropyl Trimonium Chloride | 0.02-0.2% |
| Polyquaternium-7 | 0.1-0.8% |
| Tetrasodium EDTA | 0.1-0.3% |
| Sodium Phosphate Monobasic | 0.05-0.5% |
| Sodium Lauryl Ether Sulfate | 10-15% |
| Sodium Lauryl Sulfate | 2-5% |
| Cocamidopropyl Betaine | 1-5% |
| Behenyl Alcohol | 1-3% |
| PEG-55 Propylene Glycol Oleate | 0-0.5% |
| PEG-12 Dimethicone (DC 5324) | 0.2-1.5% |
| Dimethicone 60,000 cst | 0.1-0.3% |
| Dimethyl Methyl(polypropylene oxide) siloxane (DC1248) | 0.05-0.2 |
| Preservative | 0.1-0.25% |
| Fragrance | 0.4-2.0% |

**Formulation F**

| Water | Q.S. |
|---|---|
| Polyquaternium-7 | 0.1-1% |
| Tetrasodium EDTA | 0.1-0.3% |
| Sodium Phosphate Monobasic | 0.05-0.3% |
| Ammonium Lauryl Sulfate | 6-9% |
| Ammonium Lauryl Ether Sulfate | 3-8% |
| Cocodiethanolamide | 1-3% |
| Cocomonoethanolamide | 0.5-1.5% |
| Cetyl/Stearyl Alcohol | 1-5% |
| PEG-55 Propylene Glycol Oleate | 0.02-1.5% |
| PEG-12 Dimethicone (DC 5324) | 0.2-2% |
| Dimethicone 60,000 cst | 0.05-0.2% |
| Dimethicone 350 cst | 0.1-0.4% |
| Preservative | 0.1-0.25% |
| Fragrance | 0.4-2.0% |

Examples G-I are of shampoo compositions having a combination of soluble and MQ resins and include:

**Formulation G**

| Water | Q.S. |
|---|---|
| Polyquaternium-7 | 0.2-0.5% |
| Tetrasodium EDTA | 0.05-0.1% |
| Sodium Phosphate Monobasic | 0.1-0.5% |
| Sodium Lauryl Ether Sulfate | 8-18% |
| Acrylates/steareth-20 methacrylate copolymer | 0.1-2% |
| Acrylates copolymer | 0.1-2% |
| PEG-12 Dimethicone (DC 5324) | 0.2-5% |
| Trimethylsiloxysilicate | 0.05-3% |
| Cocamidopropyl Betaine | 1-4% |
| Preservative | 0.1-0.25% |
| Fragrance | 0.4-1.0% |

**Formulation H**

| Water | Q.S. |
|---|---|
| Polyquaternium-10 | 0.1-0.5% |
| Tetrasodium EDTA | 0.1-0.2% |
| Sodium Phosphate Monobasic | 0.05-0.5% |
| Acrylates copolymer | 0.1-4% |
| Ammonium Lauryl Sulfate | 9-12% |
| PEG-12 Dimethicone (DC 5324) | 0.2-1% |
| Trimethylsiloxysilicate | 0.05-3% |
| N,N-disubstituted phtalamic acid | 0.1-2% |
| Cocodiethanolamide | 1-5% |
| Preservative | 0.1-0.25% |
| Fragrance | 0.4-2.0% |

**Formulation I**

| Water | Q.S. |
|---|---|
| Polyquaternium-7 | 0.05-1% |
| Tetrasodium EDTA | 0.1-0.2% |
| Sodium Phosphate Monobasic | 0.05-0.5% |
| Ammonium Lauryl Sulfate | 5-10% |
| Ammonium Lauryl Ether Sulfate | 3-8% |
| Acrylates/steareth-20 methacrylate copolymer | 0.1-3% |
| PEG-12 Dimethicone (DC 5324) | 0.1-5% |
| Trimethylsiloxysilicate | 0.05-3% |
| Cocomonoethanolamide | 0.5-2.5% |
| Ethylene Glycol Distearate | 0.5-5% |
| Preservative | 0.1-0.25% |
| Fragrance | 0.4-2.0% |

### EXAMPLES

Examples 1, 2, 6, 9-11, 18, 20, 21, 23, 25 and 26 are offered as illustrative of the invention and are not to be construed as limitations thereon. Examples 3, 4, 5, 7, 8, 12-17, 19, 22, 24 are not in accordance with the present invention, and are included for information. In the Examples and elsewhere in the description of the invention, chemical symbols and terminology have their usual and customary meanings. In the Examples as elsewhere in this application values for n, m, etc. in formulas, molecular weights and degree of ethoxylation or propoxylation are averages. Temperatures are in degrees C unless otherwise indicated. The amounts of the components are in weight percents based on the standard described; if no other standard is described then the total weight of the composition is to be inferred. Various names of chemical components include those listed in the CTFA International Cosmetic Ingredient Dictionary (Cosmetics, Toiletry and Fragrance Association, Inc., 7th ed. 1997).

### Examples 1-9: Translucent Shampoos with Soluble Silicones

Example 1 was made by the following procedure. In a suitable mixing vessel, water, sodium phosphate monobasic and EDTA were combined and mixed until salts were dissolved. Polquaternium-7 was added to the vessel next and mixed for 15 minutes. The sodium lauryl ether sulfate was added to the vessel and mixed for 10 minutes. Cocoamidopropyl betaine was added to the mixing vessel and mixed for 15 minutes. DC5324 was added to the vessel and mixed for 5-10 minutes, followed by the addition of the preservative to the mixing vessel. Fragrance was added to the vessel last and mixed for 10 minutes.

Examples 2-8 can be made by the same procedure as listed above for Example 1 with only varying the levels of ingredients. When used, the cocodiethanolamide can be added in place of the cocoamidopropyl betaine.

Example 9 can be made by the same procedure as listed above for Example 1 except for using DC 193 to replace DC 5324.

**TABLE A**

| **Examples** | **1** | **2** | **3** | **4** | **5** | **6** | **7** | **8** | **9** |
|---|---|---|---|---|---|---|---|---|---|
| Sodium Phosphate Monobasic | 0.2 | 0.2 | 0.2 | 0.2 | 0.2 | 0.2 | 0.2 | 0.2 | 0.2 |
| Tetrasodium EDTA | 0.2 | 0.2 | 0.2 | 0.2 | 0.2 | 0.2 | 0.2 | 0.2 | 0.1 |
| Polyquaternium-7 | 0.5 | 0.3 | 0.7 | 1.0 | 0.5 | 1.0 | 1.0 | 2.0 | 0.4 |
| Sodium Lauryl Ether Sulfate | 10 | 10 | 10 | 15 | 15 | 15 | 20 | 20 | 8 |
| Cocodiethanolamide | 0 | 0 | 0 | 3 | 0 | 0 | 5 | 0 | 0 |
| Cocamidopropyl Betaine | 2.5 | 2.5 | 2.5 | 0 | 0 | 5 | 0 | 5 | 5 |
| PEG-12 Dimethicone (DC 5324) | 0.5 | 0.5 | 0.3 | 1.5 | 2.5 | 0.5 | 3 | 5 | 0 |
| PEG-12 Dimethicone (DC 193) | 0 | 0 | 0 | 0 | 0 | 0 | 0 | 0 | 3 |
| Preservative | 0.25 | 0.25 | 0.25 | 0.25 | 0.25 | 0.2 | 0.25 | .25 | .25 |
| Fragrance | 0.5 | 0.5 | 0.5 | 1.0 | 1.0 | 1.0 | 1.5 | 1.5 | 1.5 |
| Water | Q.S. | Q.S. | Q.S. | Q.S. | Q.S. | Q.S. | Q.S | Q.S. | Q.S. |

### Examples 10-17: Opaque Shampoos with Soluble Silicones

Formula 10-17 are made based on the following procedure. Water is first added into a suitable main vessel, and stirred at 200 rpm with an overhead mixer. Ingredients in Part II is premixed with 4% water in a side vessel until uniformity achieved. Part II ingredients are added into the main vessel with continuous agitation. Start to heat the batch to 85°C. Part III ingredients are added into the main vessel one after the other, and agitation is continued for 30 minutes or until a clear solution is obtained. Meanwhile, Part IV ingredients are added into another side vessel, and heated to 85°C to melt the ingredients. When both vessels reach 85°C, transfer Part IV from the side vessel into the main vessel and increase agitation to 400 rpm. Stir the mixture at 85°C for 20 minutes, and then remove the heat. When the main vessel is at or below 38°C, the ingredients in Part V are added.

**TABLE B**

| **Examples** | **10** | **11** | **12** | **13** | **14** | **15** | **16** | **17** |
|---|---|---|---|---|---|---|---|---|
| Part I | | | | | | | | |
| Water | Q.S. | Q.S. | Q.S | Q.S. | Q.S. | Q.S. | Q.S. | Q.S. |

| Part II | | | | | | | | |
|---|---|---|---|---|---|---|---|---|
| Polyquaternium-10 | -- | -- | -- | -- | 0.25 | 0.3 | -- | -- |
| Guar Hydroxypropyl Trimonium Chloride | -- | -- | -- | -- | -- | -- | 0.1 | 0.1 |
| Polyquaternium-7 | 0.4 | 0.5 | -- | 2.0 | -- | 0.5 | 0.2 | 0.2 |
| Polyquaternium-44 | -- | -- | 3 | -- | -- | -- | -- | -- |

| Part III | | | | | | | | |
|---|---|---|---|---|---|---|---|---|
| Tetrasodium EDTA | 0.2 | 0.2 | 0.2 | 0.2 | 0.1 | 0.2 | 0.2 | 0.2 |
| Sodium Phosphate Monobasic | 0.2 | 0.1 | 0.2 | 0.2 | 0.2 | 0.2 | 0.2 | 0.2 |
| Sodium Lauryl Ether Sulfate | 8 | 15 | 6 | 17 | -- | 4 | 15 | 15 |
| Sodium Lauryl Sulfate | -- | -- | -- | -- | -- | 8 | -- | -- |
| Ammonium Lauryl Sulfate | -- | -- | -- | -- | 8 | -- | -- | -- |
| Ammonium Lauryl Ether Sulfate | -- | -- | -- | -- | 2 | -- | -- | -- |
| Lauryl/myristylamidopropyl Dimethylamine Oxide | -- | -- | -- | 2 | -- | -- | -- | -- |
| PEG-12 Dimethicone (DC 5324) | 0.4 | 0.5 | 3 | 0.5 | 0.8 | 1.5 | 2.5 | - |
| PEG-12 Dimethicone (DC 193) | -- | -- | -- | -- | -- | -- | -- | 5 |
| Cocodiethanolamide | -- | -- | -- | -- | 3 | 5 | -- | -- |
| Cocamidopropyl Betaine | 2.5 | 1.5 | 6 | -- | -- | -- | -- | -- |

| Part IV | | | | | | | | |
|---|---|---|---|---|---|---|---|---|
| Cocomonoethanolamide | -- | -- | -- | 2 | -- | -- | 4 | 4 |
| Ethylene Glycol Distearate | 1 | 2 | -- | -- | 0.4 | -- | 5 | 5 |
| Behenyl Alcohol | -- | -- | -- | 3 | 1.5 | -- | -- | -- |
| Cetyl/Stearyl Alcohol | -- | -- | 2 | -- | -- | 3 | -- | -- |
| PEG-55 Propylene Glycol Oleate | 0.1 | 0.05 | -- | -- | -- | -- | -- | -- |

| part V | | | | | | | | |
|---|---|---|---|---|---|---|---|---|
| Preservative | 0.25 | 0.25 | 0.25 | 0.2 | 0.25 | 0.25 | 0.25 | 0.25 |
| Fragrance | 0.5 | 0.5 | 1.5 | 1.0 | 0.5 | 1.0 | 1.0 | 1.0 |

### Examples 18-20: Shampoo with both Soluble and Insoluble Silicones

Formula 18-20 are made based on the following procedure. Water is first added into a suitable main vessel, and stirred at 200 rpm with an overhead mixer. Ingredients in Part II is premixed with 4% water in a side vessel until uniformity achieved. Add Part II into the main vessel with continuous agitation. Start to heat the batch to 85°C. Add ingredients in Part III into the main vessel one after the other, and keep agitating for 30 minutes or until clear solution is obtained. Meanwhile, add ingredients in Part IV into another side vessel, and heat them to 85°C to melt the ingredients. When both vessels reach 85°C, transfer Part IV from the side vessel into the main vessel and increase agitation to 400 rpm. Stir the mixture at 85°C for 20 minutes, and then remove the heat. Premix the ingredients in Part V. When the temperature of the main vessel drops to 70°C, add Part V into it with continuous agitation. When the main vessel is at or below 38°C, the ingredients in Part VI are added.

**TABLE C**

| **Examples** | **18** | **19** | **20** |
|---|---|---|---|
| Part I-Water | Q.S. | Q.S. | Q.S. |

| Part II | | | |
|---|---|---|---|
| Polyquaternium-10 | -- | 0.25 | -- |
| Guar Hydroxypropyl Trimonium Chloride | -- | -- | 0.1 |
| Polyquaternium-7 | 0.4 | -- | 0.5 |

| Part III | | | |
|---|---|---|---|
| Tetrasodium EDTA | 0.2 | 0.1 | 0.2 |
| Sodium Phosphate Monobasic | 0.2 | 0.2 | 0.1 |
| Sodium Lauryl Ether Sulfate ("SLES") | 15 | -- | 10 |
| Sodium Lauryl Sulfate | -- | -- | 5 |
| Ammonium Lauryl Sulfate | -- | 8 | -- |
| Ammonium Lauryl Ether Sulfate | -- | 4 | -- |
| Cocodiethanolamide | -- | 3 | -- |
| Cocamidopropyl Betaine | 2.5 | -- | 1.5 |

| Part IV | | | |
|---|---|---|---|
| Cocomonoethanolamide | -- | 2 | -- |
| Ethylene Glycol Distearate | -- | 0.4 | 2 |
| Behenyl Alcohol | -- | 1.5 | -- |
| Cetyl/Stearyl Alcohol | 1.5 | -- | -- |
| PEG-55 Propylene Glycol Oleate | 0.1 | -- | 0.05 |

| Part V | | | |
|---|---|---|---|
| PEG-12 Dimethicone (DC 5324) | 0.4 | 0.8 | 0.5 |
| Dimethicone 60,000 cst | 0.2 | 0.1 | |
| Dimethicone 350 cst | -- | 0.1 | -- |
| Dimethyl Methyl(polypropylene oxide) siloxane (DC1248) | -- | -- | 0.3 |

| Part VI | | | |
|---|---|---|---|
| Preservative | 0.25 | 0.25 | 0.25 |
| Fragrance | 1 | 0.8 | 2.5 |

### Examples 21-26: Shampoos with Soluble Silicone and MQ Resin

Example 21 was made by the following procedure. In a suitable making vessel, the water, sodium phosphate monobasic and EDTA was added and mixed until dissolved. The batch was heated to 65°C and maintained at the temperature. The Aculyn 22 and Aqua SF-1 were added the vessel and mixed for 5 minutes. Sodium Lauryl Ether Sulfate was added to the vessel next and mixed for 10 minutes. The pH was adjusted to between 6.5 and 7.0 with a 50% Sodium hydroxide solution. The batch was mixed for 15 minutes. The Polyquaternium-7 was pre-mixed in a side vessel with 4% water until uniform, added to the main vessel and mixed for 15 minutes. The ethylene glycol distearate was pre-melted in a side vessel with 1% SLES and heated to 65°C. When the side vessel and main vessel were both at 65°C, the side vessel that contained the EGDS was added to the main vessel and mixed for 10 minutes. The main vessel was then allowed to cool. The trimethylsiloxysilicate and DC5324 were added to the main vessel one at a time and mixed for 10 minutes. The cocamidopropyl betaine was added to the main vessel and mixed for 10 minutes. When the main vessel was at or below 38°C, the preservative and fragrance were added.

Example 22 was made by the following procedure. In a suitable making vessel, the water, sodium phosphate monobasic and EDTA was added and mixed until dissolved. The batch was heated to 80°C and maintained at the temperature. The ACULYN-22 and AQUA SF-1 were added the vessel and mixed for 5 minutes. Ammonium lauryl sulfate was added to the vessel next and mixed for 10 minutes. The pH was adjusted to between 6.5 and 7.0 with a 50% sodium hydroxide solution. The batch was mixed for 15 minutes. The Polyquaternium-7 was pre-mixed in a side vessel with 4% water until uniform, added to the main vessel and mixed for 15 minutes. The ethylene glycol distearate and cocomonoethanolamide were pre-melted in a side vessel and heated to 80°C. When the side vessel and main vessel were both at 80°C, the side vessel that contained the EGDS was added to the main vessel and mixed for 10 minutes. The main vessel was then allowed to cool. The trimethylsiloxysilicate and DC5324 materials were added to the main vessel one at a time and mixed for 10 minutes. When the main vessel was at or below 38°C, the preservative and fragrance were added.

Example 23 can be made by the same procedure as Example 21, except the Polyquaternium-10 is added in place of the Polyquaternium-7, the ACULYN-33 is added in place of the AQUA SF-1 and the TAB-2 is added in place of the EGDS.

Example 24 can be made by the same procedure as Example 21, except the Polyquaternium-10 is added in place of the Polyquaternium-7, the ACULYN-33 is added in place of the ACULYN-22 and the TAB-2 is added in place of the EGDS.

Example 25 can be made by the same procedure as Example 22, except the TAB-2 is added in place of the EGDS and the cocoamidopropyl betaine is added in place of the cocomonoethanolamide.

Example 26 can be made by the same procedure as Example 21, except without heating and omitting the EGDS premix phase.

**TABLE D**

| **Example** | **21** | **22** | **23** | **24** | **25** | **26** |
|---|---|---|---|---|---|---|
| Sodium Phosphate Monobasic | 0.2 | 0.2 | 0.2 | 0.2 | 0.2 | 0.2 |
| Tetrasodium EDTA | 0.2 | 0.2 | 0.2 | 0.2 | 0.2 | 0.2 |
| Polyquaternium-10 | 0 | 0 | 0.25 | 0.5 | 0 | 0 |
| Polyquaternium-7 | 0.35 | 0.35 | 0 | 0 | 1.0 | 0.5 |
| Aculyn-22 | 0.2 | 0.2 | 1.0 | 0 | 1.5 | 0.4 |
| Aculyn-33 | 0 | 0 | 1.0 | 1.0 | 0 | 0 |
| Aqua SF-1. | 0.2 | 0.2 | 0 | 1.0 | 1.5 | 0.4 |
| TAB-2 | 0 | 0 | 0.5 | 0.5 | 0.4 | 0 |
| Ammonium Lauryl Sulfate | 0 | 9 | 0 | 18 | 15 | 0 |
| Sodium Lauryl Ether Sulfate | 9 | 0 | 18 | 0 | 0 | 12 |
| Ethylene Glycol Distearate | 0.4 | 0.4 | 0 | 0 | 0.4 | 0 |
| Cocomonoethanolamide | 0 | 2.0 | 0 | 5 | 0 | 0 |
| Cocamidopropyl Betaine | 2.0 | 0 | 5 | 0 | 4 | 3 |
| Trimethylsiloxysilicate | 0.12 | 0.12 | 0.5 | 0.5 | 1.0 | 0.2 |
| PEG-12 Dimethicone (DC 5324) | 0.5 | 0.5 | 2.5 | 2.5 | 5 | 1.0 |
| Preservative | 0.25 | 0.25 | 0.25 | 0.25 | 0.25 | 0.25 |
| Fragrance | 0.5 | 0.5 | 1.0 | 1.5 | 1.5 | 0.75 |
| Water | Q.S. | Q.S. | Q.S. | Q.S. | Q.S. | Q.S. |

## Claims

1. A conditioning shampoo comprising:
(a) 6-19 weight % of an anionic surfactant or mixtures of two or more anionic surfactants;
(b) 0.025-3 weight % of a cationic deposition polymer, wherein the cationic deposition polymer is at least one polymer chosen from polyquatemium-7 and polyquaternium-10;
(c) 0.4-5 weight % of a detergent soluble, water dispersible silicone copolyol comprising PEG-12 dimethicone having a molecular weight of about 2300;
(d) a non-zero amount up to 5 weight % of a cocamidopropyl betaine surfactant; and
(e) water;
wherein all amounts are based on the weight of the entire composition.

2. The conditioning shampoo of claim 1 further comprising one or more of the following:
(f) 0-10 weight % of a co-surfactant, wherein the co-surfactant is at least one surfactant chosen from amphoteric surfactants, nonionic surfactants, and zwitterionic surfactants;
(g) 0-5 weight % of a pearlizer;
(h) 0-5 weight % of a salt;
(i) 0-0.5 weight % of a chelating agent;
(j) 0-1 weight % of a buffering agent;
(k) 0-5.0 weight % fragrance;
(1) 0-1 weight % of a pH adj uster;
(m) 0-5 weight % of a coloring agent;
(n) 0-5 weight % of a silicone resin;
(o) 0-10 weight % of a stabilizing agent for the silicone resin; and
(p) 0-2 weight % of a preservative.

3. The conditioning shampoo of claim 1, wherein the water content is in an amount of 57-90 weight %.

4. The conditioning shampoo of claim 1, wherein the anionic surfactant is at least one anionic surfactant selected from alkyl and alkyl ether sulfates having from 7-25 carbons; alkyl sulfuric acid salts having about 8 to about 24 carbon atoms; olefin sulfonates; beta-alkyloxy alkane sulfonates; reaction products of fatty acids esterified with isethionic acid and neutralized with sodium hydroxide; and succinamates selected from the group consisting of disodium N-octadecyl sulfofosuccinamate; tetrasodium N-(1,2-dicarboxyethyl)-N-octadecylsulfosuccinamate; diamyl eater of sodium sulfosuccinic acid; dihexyl ester of sodium sulfosuccinic acid; and dioctyl esters of sodium sulfosuccinic acid.

5. The conditioning shampoo of claim 1, wherein the anionic surfactant is at least one anionic surfactant selected from ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, monoethanolamine cocoyl sulfate, monoethanolamine lauryl sulfate, sodium tridecyl benzene sulfonate, and sodium dodecyl benzene sulfonate.

6. The conditioning shampoo ofclaim 1 further comprising a nonionic surfactant, wherein the nonionic surfactant is at least one surfactant chosen from:
(i) polyethylene oxide condensates of alkyl phenols wherein the alkyl portion of the alkyl phenol has 6-12 carbons and may be straight chain or branched and the ethylene oxide portion is present in an amount of 10-60 moles of ethylene oxide per mole of alkyl phenol;
(ii) condensation products of ethylene oxide with a product resulting from the reaction of propylene oxide and ethylene diamine;
(iii) condensation products of C₈-₁₈ straight or branched chain aliphatic alcohols with ethylene oxide;
(iv) long chain tertiary amine oxides of formula (R¹)(R²)(R³)-N→ O,
wherein R¹ is an C₈-1₈ alkyl, alkenyl or monohydroxy alkyls; which has from 0-10 ethylene moieties and from 0-1 glyceryl moiety; and R² and R³ may be the same or different and are each independently selected from the group consisting of C₁-₃ alkyls with 0-1 hydroxy group;
(v) long chain tertiary phosphine oxides of Formula: R⁴ R⁵ R⁶ P→ O where R⁴ contains a C₈-₁₈ alkyl, alkenyl or monohydroxyalkyl radical; 0-10 ethylene oxide moieties and 0-1 glyceryl moiety; and R⁵ and R⁶ are each independently C₁-₃ alkyl or monohydroxyalkyl; and
(vi) long chain dialkyl sulfoxides containing one short chain alkyl or hydroxy alkyl radical of 1-3 carbons and one long hydrophobic chain having a C₈-₂₀ alkyl, alkenyl, hydroxy alkyl or keto alkyl group, with 0-10 ethylene oxide moieties and 0-1 glyceryl moiety.

7. The conditioning shampoo of claim 2, wherein the co-surfactant is present.

8. The conditioning shampoo of claim 7, wherein the co-surfactant comprises a nonionic surfactant, wherein the nonionic surfactant is at least one surfactant chosen from esters of polyols or sugars; fatty acid alkanolamides having 7-25 carbons; pyrrolidones; and the condensation products of ethylene oxide and C₇-C₂₅ long chain amides.

9. The conditioning shampoo of claim 8, wherein the alkanolamide is at least one alkanolamide chosen from cocamide MEA, cocamide DEA, soyamide DEA, lauramide DEA, oleamide MIPA, stearamide MEA, myristamide MEA, lauramide MEA, capramide DEA, ricinoleamide DEA, myristamide DEA, stearamide DEA, oleylamide DEA, tallowamide DEA, lauramide MEA, tallowamide MEA, isostearamide DEA, and isostearamide MEA, where DEA means diethanolamide; MEA means monoethanolamide; and MIPA means monoisopropyl amide.

10. The conditioning shampoo of claim 7 further comprising an amphoteric surfactant, wherein the amphoteric surfactant is at least one surfactant chosen from aliphatic secondary and tertiary amines in which the aliphatic substituent contains from about 8 to 18 carbon atoms and contains an anionic water solubilizing group selected from the group consisting of carboxy, sulfonate, sulfate, phosphate, and phosphonate.

11. The conditioning shampoo of claim 10, wherein the amphoteric surfactant is at least one surfactant chosen from sodium 3-dodecyl-aminopropionate, sodium 3-dodecylaminopropane sulfonate, sodium lauryl sarcosinate, N-alkyltaurines, and N-alkyl aspartic acids.

12. The conditioning shampoo of claim 1, wherein the cationic deposition polymer comprises polyquatemium-7.

13. The conditioning shampoo of claim 1, wherein the cationic deposition polymer comprises polyquaternium-10.

14. The conditioning shampoo of claim 1, wherein the cationic deposition polymer is present in an amount of 0.05 to 3 weight %.

15. The conditioning shampoo of claim 1, wherein the cationic deposition polymer is present in an amount of 0.1 to 3 weight %.

16. The conditioning shampoo of claim 2, wherein the salt is NaCl.

17. The conditioning shampoo of claim 1 further comprising an MQ resin.

## Patentansprüche

1. Konditionierendes Shampoo, umfassend:
(a) 6-19 Gew.% eines anionischen Tensids oder Mischungen von zwei oder mehreren anionischen Tensiden;
(b) 0,025-3 Gew.% eines kationischen Abscheidungspolymers, wobei das kationische Abscheidungspolymer mindestens ein Polymer ist, das aus Polyquaternium-7 und Polyquaternium-10 ausgewählt ist;
(c) 0,4-5 Gew.% eines in Detergens löslichen, in Wasser dispergierbaren Silikon-Copolyols, das PEG-12-Dimethicon mit einem Molekulargewicht von etwa 2300 umfasst;
(d) eine Menge, die unlgeich null ist und bis zu 5 Gew.% eines Cocamidpropylbetain-Tensids und
(e) Wasser;
wobei alle Mengenangaben auf Basis des Gewichts der gesamten Zusammensetzung basieren.

2. Konditionierendes Shampoo nach Anspruch 1, das weiterhin eines oder mehrere der Folgenden umfasst:
(f) 0-10 Gew.% eines Cotensids, wobei das Cotensid mindestens ein Cotensid ist, das aus der Gruppe bestehend aus amphoteten Tensiden, nichtionischen Tensiden und zwitterionischen Tensiden ausgewählt ist;
(g) 0-5 Gew.% eines Pearlizers;
(h) 0-5 Gew.% eines Salzes;
(i) 0-0,5 Gew.% eines Chelatisierungsmittels;
(j) 0-1 Gew.% eines Puffers;
(k) 0-5,0 Gew.% eines Duftstoffes;
(1) 0-1 Gew.% eines pH-Wert-Einstellungsmittels;
(m) 0-5 Gew.% eines Farbstoffes;
(n) 0-5 Gew.% eines Silikonharzes;
(o) 0-10 Gew.% eines Stabilisierungsmittels für das Silikonharz und
(p) 0-2 Gew.% eines Konservierungsmittels.

3. Konditionierendes Shampoo nach Anspruch 1, wobei der Wassergehalt 57-90 Gew.% beträgt.

4. Konditionierendes Shampoo nach Anspruch 1, wobei das anionische Tensid aus der Gruppe bestehend aus Alkyl- und Alykl-Ether-Sulfaten mit 7 bis 25 Kohlenstoffatomen; Alkylschwefelsäuresalzen mit etwa 8 bis 24 Kohlenstofftomen; Olefinsulfonaten; Betaalkoxyalkansulfonaten; Reaktionsprodukten von mit Isethionsäure veresterten und mit Natriumhydroxid neutralisierten Fettsäuren und Succinamaten ausgewählt ist, die aus der Gruppe bestehend aus Dinatrium-N-octadecylsulfosuccinamaten, Tetranatrium-N-(1,2-dicarboxyethyl)-N-octadecylsulfosuccinamaten; Diamylester von Natriumsulfosuccinat; Dihexylester von Natriumsulfosuccinat und Dioctylestern von Natriumsulfosuccinat ausgewählt sind.

5. Konditionierendes Shampoo nach Anspruch 1, wobei das anionische Tensid mindestens ein anionisches Tensid ist, das aus Ammoniumlaurylsulfat, Ammoniumlaurethsulfat, Triethylaminlaurylsulfat, Triethylaminlaurethsulfat, Triethanolaminlaurylsulfat, Triethanolaminlaurethsulfat, Monoethaolaminlaurylsulfat, Monoethanolaminlaurethsulfat, Diethanolaminlaurylfulfat, Diethanolaminlaurethsulfat, Lauryl-Monoglycerid-Natriumsulfat, Natriumlaurylsulfat, Natriumlaurethsulfat, Kaliumlaurylsulfat, Kaliumlaurethsulfat, Natriumlaurylsarcosinat, Natriumlauroylsarcosinat, Laurylsarcosin, Cocoylsarcosin, Ammoniumcocoylsulfat, Ammoniumlauroylsulfat, Kaliumcocoylsulfat, Kaliumlauroylsulfat, Triethanolaminlaurylsulfat, Monoethanolamincocoylsulfat, Monoethanolaminlaurylsulfat, Natriumtridecylbenzolsulfonat und Natriumdodecylbenzolsulfonat ausgewählt ist.

6. Konditionierendes Shampoo nach Anspruch 1, das weiterhin ein nichtionisches Tensid umfasst, wobei das nichtionische Tensid mindestens ein Tensid ist, das aus:
(i) Polyethylenoxidkondensat von Alkylphenolen, wobei der Alkylanteil des Aklylphenols 6-12 Kohlenstoffatome aufweist und geradkettig oder verzweigt sein kann und der Ethylenoxidanteil in einer Menge von 10-60 Molen Ethylenoxid pro Mol Alkylphenol vorhanden ist;
(ii) Kondensationsprodukten von Ethylenoxid mit einem Produkt, das aus der Reaktion von Propylenoxid und Ethylendiamin resultiert;
(iii) Kondensationsprodukten von geradkettigen oder verzweigtkettigen aliphatischen C₈₋₁₈-Alkoholen mit Ethylenoxid;
(iv) langkettigen tertiären Aminoxiden der Formel (R¹) (R²) (R³) -N→O, wobei R¹ ein C₈₋₁₈-Alkyl, -Alkenyl oder Monohydroxyalkyl ist, das 0-10 Ethyleneinheiten und 0-1 Glycerineinheiten aufweist; und R² und R³ gleich oder unterschiedlich sind und jeweils unabhängig aus der Gruppe bestehend aus C₁₋₃-Alkylen mit 0-1 Hydroxygruppen ausgewählt sind;
(v) langkettigen tertiären Phosphinoxiden der Formel R⁴R⁵R⁶-P→O, wobei R⁴ ein C₈₋₁₈-Alkyl, -Alkenyl oder Monohydroxyalkylrest ist, der 0-10 Ethyleneinheiten und 0-1 Glycerineinheiten aufweist; und R⁵ und R⁶ jeweils unabhängig C₁₋₃-Alkyl oder Monohydroxyalkyl sind; und
(vi) langkettigen Dialkylsulfoxiden ausgewählt ist, die einen kurzkettigen Alkyl- oder Hydroxyalkylrest mit 1-3 Kohlenstoffatomen und eine lange hydrophoben-Kette mit einer C₈₋₂₀-Alkyl-, Alkenyl-, Hydroxyalkyl- oder Ketoalkylgruppe mit 0-10 Ethylenoxideinheiten und 0-1 Glycerineinheit enthalten.

7. Konditionierendes Shampoo nach Anspruch 2, wobei Cotensid vorhanden ist.

8. Konditionierendes Shampoo nach Anspruch 7, wobei das Cotensid ein nichtionisches Tensid umfasst, wobei das nichtionische Tensid mindestens ein Tensid ist, das aus Estern von Polyolen oder Zuckern, Fettsäurealkanolaminen mit 7-25 Kohlenstoffatomen, Pyrrolidonen und den Kondensationsprodukten von Ethylenoxid und langkettigen C₇-C₂₅ Amiden ausgewählt ist.

9. Konditionierendes Shampoo nach Anspruch 8, wobei das Alkanolamid mindestens ein Alkanolamid ist, das aus der Gruppe bestehend aus Cocamid-MEA, Cocamid-DEA, Soyamid-DEA, Lauramid-DEA, Oleamid-MIPA, Stearamid-MEA, Myristinamid-MEA, Lauramid-MEA, Capramid-MEA, Ricinolamid-DEA, Myristinamid-DEA, Stearamid-DEA, Oleylamid-DEA, Talkamid-DEA, Lauramid-MEA, Talkamid-MEA, Isostearamid-DEA und Isostearamid-MEA ausgewählt ist, wobei DEA Diethanolamid, MEA Monoethanolamid und MIPA Monoisopropylamid bedeuten.

10. Konditionierendes Shampoo nach Anspruch 7, das weiterhin ein amphoteres Tensid umfasst, wobei das amphotere Tensid mindestens ein Tensid ist, das aus aliphatischen sekundären und tertiären Aminen ausgewählt ist, in denen der aliphatische Substituent etwa 8 bis 18 Kohlenstoffatome und eine anionische wasserlösliche Gruppe enthält, die aus der Gruppe bestehend aus Carboxy, Sulfonat, Sulfat, Phosphat und Phosphonat ausgewählt ist.

11. Konditionierendes Shampoo nach Anspruch 10, wobei das amphotere Tensid mindestens ein Tensid ist, das aus Natrium-3-dodecylaminoproprionat, Natrium-3-dodecylaminopropansulfonat, Natriumlaurylsarcosinat, N-Alkyltaurinen und N-Alkylaspartamsäure ausgewählt ist.

12. Konditionierendes Shampoo nach Anspruch 1, wobei das kationische Abscheidungspolymer Polyquarternium-7 umfasst.

13. Konditionierendes Shampoo nach Anspruch 1, wobei das kationische Abscheidungspolymer Polyquarternium-10 umfasst.

14. Konditionierendes Shampoo nach Anspruch 1, wobei das kationische Abscheidungspolymer in einer Menge von 0,05 bis 3 Gew.% vorhanden ist.

15. Konditionierendes Shampoo nach Anspruch 1, wobei das kationische Abscheidungspolymer in einer Menge von 0,1 bis 3 Gew.% vorhanden ist.

16. Konditionierendes Shampoo nach Anspruch 2, wobei das Salz NaCl ist.

17. Konditionierendes Shampoo nach Anspruch 1, das weiterhin ein MQ-Harz umfasst.

## Revendications

1. Shampooing traitant comprenant :
(a) de 6 à 19 % en poids d'un tensioactif anionique ou de mélanges de deux ou plusieurs tensioactifs anioniques ;
(b) de 0,025 à 3 % en poids d'un polymère de dépôt cationique, dans lequel le polymère de dépôt cationique est au moins un polymère choisi parmi le polyquatemium-7 et le polyquatemium-10 ;
(c) de 0,4 à 5 % en poids d'un copolyol de silicone soluble dans les détergents et dispersible dans l'eau, comprenant de la PEG-12 diméthicone présentant un poids moléculaire d'environ 2 3 00 ;
(d) une quantité non nulle pouvant atteindre 5 % en poids d'un tensioactif de cocamidopropylbétaïne ; et
(e) de l'eau ;
dans lequel toutes les quantités sont basées sur le poids de la composition totale.

2. Shampooing traitant selon la revendication 1 comprenant en outre un ou plusieurs des éléments suivants :
(f) de 0 à 10 % en poids d'un co-tensioactif, dans lequel le co-tensioactif est au moins un tensioactif choisi parmi les tensioactifs amphotères, les tensioactifs non ioniques et les tensioactifs zwitterioniques ;
(g) de 0 à 5 % en poids d'un agent nacrant ;
(h) de 0 à 5 % en poids d'un sel ;
(i) de 0 à 0,5 % en poids d'un agent chélateur ;
(j) de 0 à 1 % en poids d'un agent tampon ;
(k) de 0 à 5,0 % en poids d'un parfum ;
(1) de 0 à 1 % en poids d'un agent d'ajustement du pH ;
(m) de 0 à 5 % en poids d'un colorant ;
(n) de 0 à 5 % en poids d'une résine de silicone ;
(o) de 0 à 10 % en poids d'un agent stabilisant pour la résine de silicone ; et
(p) de 0 à 2 % en poids d'un agent de conservation.

3. Shampooing traitant selon la revendication 1, dans lequel la teneur en eau est présente en une quantité allant de 57 à 90 % en poids.

4. Shampooing traitant selon la revendication 1, dans lequel le tensioactif anionique est au moins un tensioactif anionique choisi parmi les sulfates d'alkyle et d'alkyléther comportant de 7 à 25 atomes de carbone ; les sels d'acide alkylsulfurique comportant environ 8 à environ 24 atomes de carbone ; les sulfonates d'oléfine ; les sulfonates de bêta-alkyloxyalcane ; les produits de réaction des acides gras estérifiés avec de l'acide iséthionique et neutralisés avec de l'hydroxyde de sodium ; et les succinamates choisis parmi le groupe comprenant du sulfosuccinamate de N-octadécyle disodique ; du N-(1,2-dicarboxyéthyl)-N-octadécylsulfosuccinamate de tétrasodium ; de l'ester diamylique de l'acide sulfosuccinique sodique ; de l'ester dihexylique de l'acide sulfosuccinique sodique ; et des esters dioctyliques de l'acide sulfosuccinique sodique.

5. Shampooing traitant selon la revendication 1, dans lequel le tensioactif anionique est au moins un tensioactif anionique choisi parmi le laurylsulfate d'ammonium, le laureth sulfate d'ammonium, le laurylsulfate de triéthylamine, le laureth sulfate de triéthylamine, le laurylsulfate de triéthanolamine, le laureth sulfate de triéthanolamine, le laurylsulfate de monoéthanolamine, le laureth sulfate de monoéthanolamine, le laurylsulfate de diéthanolamine, le laureth sulfate de diéthanolamine, le sulfate de sodium de monoglycéride laurique, le laurylsulfate de sodium, le laureth sulfate de sodium, le laurylsulfate de potassium, le laureth sulfate de potassium, le laurylsarcosinate de sodium, le lauroylsarcosinate de sodium, la laurylsarcosine, la cocoylsarcosine, le cocoylsulfate d'ammonium, le lauroylsulfate d'ammonium, le cocoylsulfate de sodium, le lauroylsulfate de sodium, le cocoylsulfate de potassium, le laurylsulfate de potassium, le laurylsulfate de triéthanolamine, le cocoylsulfate de monoéthanolamine, le laurylsulfate de monoéthanolamine, le tridécylbenzènesulfonate de sodium et le dodécylbenzènesulfonate de sodium.

6. Shampooing traitant selon la revendication 1 comprenant en outre un tensioactif non ionique, dans lequel le tensioactif non ionique est au moins un tensioactif choisi parmi :
(i) les condensats d'oxyde de polyéthylène d'alkylphénols dans lesquels la partie alkyle de l'alkylphénol comporte de 6 à 12 atomes de carbone et peut être à chaîne linéaire ou ramifiée et la partie oxyde d'éthylène est présente en une quantité de 10 à 60 moles d'oxyde d'éthylène par mole d'alkylphénol ;
(ii) les produits de condensation de l'oxyde d'éthylène avec un produit résultant de la réaction de l'oxyde de propylène et de l'éthylènediamine ;
(iii) les produits de condensation des alcools aliphatiques en C₈ à C₁₈ à chaîne linéaire ou ramifiée avec de l'oxyde d'éthylène ;
(iv) les oxydes d'amine tertiaire à chaîne longue de formule (R¹)(R²)(R³)-N → O, où R¹ représente un groupe alkyle, alcényle ou monohydroxyalkyle en C₈ à C₁₈ ; qui comporte de 0 à 10 groupements éthylène et de 0 à 1 groupement glycéryle ; et R² et R³ peuvent être identiques ou différents et sont chacun indépendamment choisis dans le groupe comprenant des groupes alkyles en C₁ à C₃ comportant de 0 à 1 groupe hydroxy ;
(v) les oxydes de phosphine tertiaire à chaîne longue de formule : R⁴ R⁵ R⁶ P → O où R⁴ contient un radical alkyle, alcényle ou monohydroxyalkyle en C₈ à C₁₈ ; de 0 à 10 groupements oxyde d'éthylène et de 0 à 1 groupement glycéryle ; et R⁵ et R⁶ représentent chacun indépendamment un groupe alkyle ou monohydroxyalkyle en C₁ à C₃ ; et
(vi) les sulfoxydes de dialkyle à chaîne longue contenant un radical alkyle ou hydroxyalkyle à chaîne courte de 1 à 3 atomes de carbone et une longue chaîne hydrophobe comportant un groupe alkyle, alcényle, hydroxyalkyle ou cétoalkyle en C₈ à C₂₀, comportant de 0 à 10 groupements oxyde d'éthylène et de 0 à 1 groupement glycéryle.

7. Shampooing traitant selon la revendication 2, dans lequel le co-tensioactif est présent.

8. Shampooing traitant selon la revendication 7, dans lequel le co-tensioactif comprend un tensioactif non ionique, dans lequel le tensioactif non ionique est au moins un tensioactif choisi parmi les esters de polyols ou de sucres ; les alcanolamides d'acides gras comportant de 7 à 25 atomes de carbone ; les pyrrolidones ; et les produits de condensation d'oxyde d'éthylène et d'amides à chaîne longue en C₇ à C₂₅.

9. Shampooing traitant selon la revendication 8, dans lequel l'alcanolamide est au moins un alcanolamide choisi parmi le cocamide MEA, le cocamide DEA, le soyamide DEA, le lauramide DEA, l'oléamide MIPA, le stéaramide MEA, le myristamide MEA, le lauramide MEA, le capramide DEA, le ricinoléamide DEA, le myristamide DEA, le stéaramide DEA, l'oléylamide DEA, l'amide de suif DEA, le lauramide MEA, l'amide de suif MEA, l'isostéaramide DEA et l'isostéaramide MEA, où DEA signifie diéthanolamide ; MEA signifie monoéthanolamide ; et MIPA signifie monoisopropylamide.

10. Shampooing traitant selon la revendication 7 comprenant en outre un tensioactif amphotère, dans lequel le tensioactif amphotère est au moins un tensioactif choisi parmi les amines aliphatiques secondaires et tertiaires dans lesquelles le substituant aliphatique contient environ 8 à 18 atomes de carbone et contient un groupe anionique solubilisant dans l'eau choisi parmi le groupe comprenant du carboxy, du sulfonate, du sulfate, du phosphate et du phosphonate.

11. Shampooing traitant selon la revendication 10, dans lequel le tensioactif amphotère est au moins un tensioactif choisi parmi le 3-dodécyl-aminopropionate de sodium, le 3-dodécylaminopropanesulfonate de sodium, le laurylsarcosinate de sodium, les N-alkyltaurines et les acides N-alkylaspartiques.

12. Shampooing traitant selon la revendication 1, dans lequel le polymère de dépôt cationique comprend du polyquatemium-7.

13. Shampooing traitant selon la revendication 1, dans lequel le polymère de dépôt cationique comprend du polyquaternium-10.

14. Shampooing traitant selon la revendication 1, dans lequel le polymère de dépôt cationique est présent en une quantité allant de 0,05 à 3 % en poids.

15. Shampooing traitant selon la revendication 1, dans lequel le polymère de dépôt cationique est présent en une quantité allant de 0,1 à 3 % en poids.

16. Shampooing traitant selon la revendication 2, dans lequel le sel est du NaCl.

17. Shampooing traitant selon la revendication 1 comprenant en outre une résine MQ.
